# EUROPEAN PATENT APPLICATION

(11) **EP 4 714 448 A1**
(43) Date of publication of application: **25.03.2026**
(21) Application number: 24792136.4
(22) Date of filing: 19.04.2024
(51) Int. Cl.: A61K 31/496, A61K 31/404, C07D 471/04, C07D 211/96, A61P 35/00, A61P 35/02

(54) **USE OF TRIFLUOROMETHYL-CONTAINING COMPOUND IN TREATMENT OF HEMATOLOGICAL TUMORS**

(30) Priority: 20.04.2023 CN 202310426190; 20.04.2023 CN 202310429276
(71) Applicant: Chia Tai Tianqing Pharmaceutical Group Co., Ltd., Lianyungang, Jiangsu 222062 (CN)
(72) Inventor: ZHAO, Qian, Lianyungang, Jiangsu 222062 (CN); ZHANG, Xiquan, Lianyungang, Jiangsu 222062 (CN); ZHAO, Wei, Lianyungang, Jiangsu 222062 (CN); WANG, Xunqiang, Lianyungang, Jiangsu 222062 (CN); YU, Ding, Lianyungang, Jiangsu 222062 (CN); ZHANG, Qianqian, Lianyungang, Jiangsu 222062 (CN)
(74) Representative: WSL Patentanwälte Partnerschaft mbB
(86) International application number: PCT/CN2024/088858
(87) International publication number: WO 2024/217552

(57) **Abstract**

The present application belongs to the field of medicine, relates to the use of a trifluoromethyl-containing compound in the treatment of hematological tumors, and specifically relates to the use of a compound of formula I-1 or a pharmaceutically acceptable salt thereof in the treatment of hematological tumors

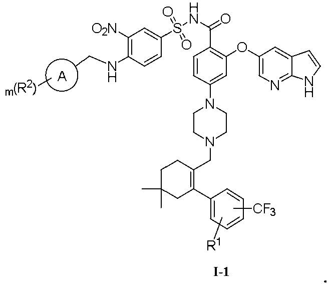

## Description

### CROSS-REFERENCE TO RELATED APPLICATIONS

The present application claims priority and benefit to the Chinese Patent Application No. 202310429276.0 filed with China National Intellectual Property Administration on April 20, 2023, and the Chinese Patent Application No. 202310426190.2 filed with China National Intellectual Property Administration on April 20, 2023, which are incorporated herein by reference in their entirety.

### TECHNICAL FIELD

The present application pertains to the field of medicine, relates to use of a trifluoromethyl-containing compound for treating a hematologic tumor, and specifically relates to use of a compound of formula I-1 or a pharmaceutically acceptable salt thereof for treating a hematologic tumor.

### BACKGROUND

BCL-2 proteins are classified into three families: the BCL-2 family (whose members include BCL-2, BCL-XL, etc.), the BAX family, and the BH3-only family. Among them, the BCL-2 family plays an anti-apoptotic role, while members of the latter two families play a pro-apoptotic role.

Anti-apoptotic proteins of the BCL-2 family are associated with many diseases and are being investigated as potential targets of therapeutic agents. These targets for interventional therapy include, for example, the BCL-2 family proteins such as BCL-2 and BCL-XL. Inhibitors of the BCL-2 family proteins are inhibitors that bind to a target protein, but the binding affinity of the compounds is only one of many parameters to be considered. One objective is to produce a compound that preferentially binds to, i.e., has selectivity for, one protein over another. The manifestation of this selectivity, as is well known, is the compound's high binding affinity for a specific protein and lower binding affinity for another.

Compounds that selectively inhibit the BCL-2 protein can be used for treating hyperproliferative diseases, such as tumors. For example, WO2019185025, WO2020088442, and WO2020238785 disclose compounds as BCL-2 inhibitors or pharmaceutically acceptable salts thereof.

### SUMMARY

In one aspect, the present application provides a method for treating a hematologic tumor, comprising administering to a subject a therapeutically effective amount of a BCL-2 inhibitor or a pharmaceutically acceptable salt thereof, or a pharmaceutical composition thereof.

In another aspect, the present application provides use of a BCL-2 inhibitor or a pharmaceutically acceptable salt thereof, or a pharmaceutical composition thereof for preparing a medicament for treating a hematologic tumor. The present application further provides a BCL-2 inhibitor or a pharmaceutically acceptable salt thereof, or a pharmaceutical composition thereof for use in treating a hematologic tumor. The present application further provides use of the BCL-2 inhibitor or the pharmaceutically acceptable salt thereof, or the pharmaceutical composition thereof of the present application for treating a hematologic tumor.

In yet another aspect, the present application further provides a pharmaceutical composition for use in treating a hematologic tumor, comprising a BCL-2 inhibitor or a pharmaceutically acceptable salt thereof as an active ingredient.

In some embodiments, the present application further provides a kit for use in treating a hematologic tumor, comprising: a pharmaceutical composition comprising a BCL-2 inhibitor or a pharmaceutically acceptable salt thereof, and optionally further comprising an instruction for use of the pharmaceutical composition of the BCL-2 inhibitor or the pharmaceutically acceptable salt thereof. In some embodiments, the instruction for use guides the administration of the pharmaceutical composition comprising the BCL-2 inhibitor or the pharmaceutically acceptable salt thereof to a subject.

In some embodiments, the BCL-2 inhibitor or the pharmaceutically acceptable salt thereof described in the present application is selected from a compound of formula I-1 or a pharmaceutically acceptable salt thereof, 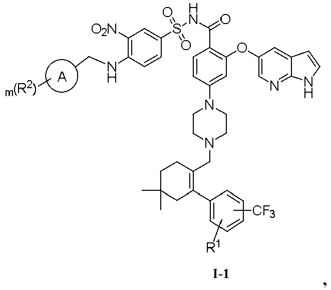 wherein
R¹ is selected from the group consisting of hydrogen, halogen, and C₁₋₆ alkyl;
ring A is selected from the group consisting of 5- to 6-membered heterocycloalkyl;
each R² is independently selected from the group consisting of 4- to 6-membered heterocycloalkyl, C₃₋₆ cycloalkyl, -COR^{a}, -SO₂R^{b}, and C₁₋₆ alkyl optionally substituted with halogen;
each R^{a} or R^{b} is independently selected from the group consisting of H, 4- to 6-membered heterocycloalkyl, C₃₋₆ cycloalkyl, and C₁₋₆ alkyl, wherein the C₁₋₆ alkyl is optionally substituted with halogen, -CN, -N(C₁₋₆ alkyl)₂, -NHC₁₋₆ alkyl, or -OC₁₋₆ alkyl;
m is selected from the group consisting of 0, 1, 2, and 3.

In some embodiments, the structural fragment 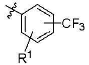 is selected from

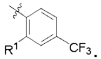

In some embodiments, R¹ is selected from the group consisting of hydrogen, halogen, and C₁₋₃ alkyl. In some embodiments, R¹ is selected from the group consisting of hydrogen, fluorine, chlorine, and methyl. In some embodiments, R¹ is selected from the group consisting of hydrogen, chlorine, and methyl.

In some embodiments, ring A is selected from 6-membered heterocycloalkyl. In some embodiments, ring A is selected from 6-membered heterocycloalkyl containing one or more O atoms, for example, 6-membered heterocycloalkyl containing 1 or 2 O atoms. In some embodiments, ring A is selected from the group consisting of dioxane and a pyran ring.

In some embodiments, each R² is independently selected from the group consisting of 4- to 6-membered heterocycloalkyl, C₄₋₆ cycloalkyl, -COR^{a}, -SO₂R^{b}, and C₁₋₄ alkyl optionally substituted with halogen.

In some embodiments, each R^{a} or R^{b} is independently selected from the group consisting of H, 4- to 6-membered heterocycloalkyl, C₃₋₆ cycloalkyl, and C₁₋₄ alkyl, wherein the C₁₋₄ alkyl is optionally substituted with halogen, -CN, -N(C₁₋₄ alkyl)₂, -NHC₁₋₄ alkyl, or -OC₁₋₄ alkyl.

In some embodiments, each R² is independently selected from the group consisting of -C(O)H, -COC(CH₃)₃, - COCF₃, -COCH₂CN, -COCH₂N(CH₃)₂, -SO₂CH₂CH₃, -SO₂CF₃, -SO₂C₂F₅, -CF₃, -C₂F₅, tetrahydropyran, monooxetane, -SO₂-cyclopropane, -CO-cyclopropane, -CO-monooxetane, -SO₂-monooxetane, and -SO₂-cyclobutane.

In some embodiments, m is selected from the group consisting of 0 and 1.

In some embodiments, the BCL-2 inhibitor or the pharmaceutically acceptable salt thereof described in the present application is selected from the group consisting of the compounds of formula I, formula II, formula III, and formula IV, or pharmaceutically acceptable salts thereof,

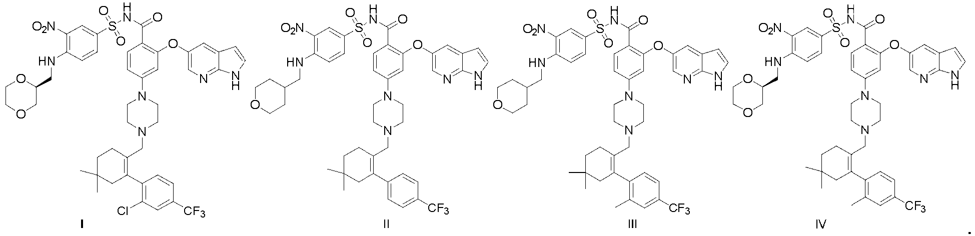

In some embodiments, the BCL-2 inhibitor or the pharmaceutically acceptable salt thereof is selected from the compound of formula I or a pharmaceutically acceptable salt thereof, or selected from the compound of formula II or a pharmaceutically acceptable salt thereof, or selected from the compound of formula III or a pharmaceutically acceptable salt thereof, or selected from the compound of formula IV or a pharmaceutically acceptable salt thereof. In some embodiments, the compound of formula I-1 or the pharmaceutically acceptable salt thereof is selected from the compound of formula I or a pharmaceutically acceptable salt thereof. In some embodiments, the compound of formula I-1 or the pharmaceutically acceptable salt thereof is selected from the compound of formula II or a pharmaceutically acceptable salt thereof. In some embodiments, the compound of formula I-1 or the pharmaceutically acceptable salt thereof is selected from the compound of formula III or a pharmaceutically acceptable salt thereof. In some embodiments, the compound of formula I-1 or the pharmaceutically acceptable salt thereof is selected from the compound of formula IV or a pharmaceutically acceptable salt thereof.

### Hematologic Tumor

In some embodiments, the hematologic tumor is selected from an advanced hematologic tumor. In some embodiments, the hematologic tumor is selected from a recurrent and/or refractory hematologic tumor. In some embodiments, the hematologic tumor is selected from a recurrent and/or refractory advanced hematologic tumor.

In some embodiments, the hematologic tumor is selected from a hematologic tumor definitively diagnosed by histology or cytology. In some embodiments, the diagnosis described above meets the 2016 WHO diagnostic criteria. In some embodiments, the hematologic tumor is selected from the group consisting of a lymphoid tumor and leukemia.

In some embodiments, the hematologic tumor is selected from the group consisting of a lymphoid tumor and myeloid leukemia. In some embodiments, the lymphoid tumor is selected from non-Hodgkin's lymphoma. In some embodiments, the myeloid leukemia is acute myeloid leukemia.

In some embodiments, the hematologic tumor is selected from the group consisting of non-Hodgkin's lymphoma and acute myeloid leukemia. In some embodiments, the non-Hodgkin's lymphoma is selected from the group consisting of mantle cell lymphoma, diffuse large B-cell lymphoma, chronic lymphocytic leukemia, and/or small lymphocytic lymphoma.

In some embodiments, the hematologic tumor is selected from the group consisting of mantle cell lymphoma, diffuse large B-cell lymphoma, chronic lymphocytic leukemia and/or small lymphocytic lymphoma, and acute myeloid leukemia.

In some embodiments, the hematologic tumor is selected from leukemia.

In some embodiments, the hematologic tumor is selected from myeloid leukemia.

In some embodiments, the hematologic tumor or leukemia is selected from acute myeloid leukemia.

In some embodiments, the hematologic tumor is selected from a lymphoid tumor.

In some embodiments, the hematologic tumor is selected from non-Hodgkin's lymphoma.

In some embodiments, the hematologic tumor or non-Hodgkin's lymphoma is selected from the group consisting of mantle cell lymphoma, diffuse large B-cell lymphoma, chronic lymphocytic leukemia, and/or small lymphocytic lymphoma.

In some embodiments, the subject with the hematologic tumor may be a subject who has not received treatment with a prior therapeutic regimen. In some embodiments, the subject with the hematologic tumor has received treatment with one or two or more prior therapeutic regimens. In some embodiments, the subject with the hematologic tumor has received treatment with one, two, three, four, or five prior therapeutic regimens.

In some embodiments, the subject with the hematologic tumor is selected from a subject with a recurrent or refractory disease. In some embodiments, the subject with the hematologic tumor is selected from a subject with a disease refractory to initial induction or re-induction therapy. In some embodiments, the subject with the hematologic tumor is selected from a subject who has experienced recurrence after a previous treatment or has failed a previous treatment. In some embodiments, the subject with the hematologic tumor is selected from a subject who has experienced recurrence after a previous standard treatment or has failed a previous standard treatment. In some embodiments, the subject with the hematologic tumor is selected from a subject who has failed a previous treatment. In some embodiments, the subject with the hematologic tumor is selected from a subject who has failed a standard treatment. In some embodiments, the subject with the hematologic tumor is selected from a subject who is intolerant to a previous therapeutic regimen. In some embodiments, the subject with the hematologic tumor is selected from a subject who is intolerant to a standard therapeutic regimen. In some embodiments, the subject with the hematologic tumor is selected from a subject who is intolerant to a previous therapeutic regimen and has no other better treatment options. In some embodiments, the subject with the hematologic tumor is selected from a subject who is intolerant to a standard therapeutic regimen and has no other better treatment options.

In some embodiments, the subject with the hematologic tumor is selected from a subject who has previously received at least 1 systemic therapeutic regimen, and has experienced disease progression or intolerance during or after the most recent treatment, or has not achieved a response after adequate treatment. In some embodiments, the subject with the hematologic tumor is selected from a subject who has previously received at least 1 or 2 systemic therapeutic regimens, and has experienced disease progression or intolerance during or after the most recent treatment, or has not achieved a response after adequate treatment.

In some embodiments, the previously received therapeutic regimen includes a first-line therapeutic regimen, a second-line therapeutic regimen, and/or a third-line therapeutic regimen.

In some embodiments, the subject with the hematologic tumor has at least 1 efficacy-evaluable lesion/measurable disease.

In some embodiments, the subject with the acute myeloid leukemia has bone marrow blast cells. In some embodiments, the subject with the acute myeloid leukemia has ≥ 5% bone marrow blast cells.

In some embodiments, the subject with the non-Hodgkin's lymphoma has at least one radiologically measurable tumor lesion (e.g., the longest diameter of a nodal lesion > 15 mm, and the longest diameter of an extranodal lesion > 10 mm) in 2 perpendicular directions as assessed by CT or MRI.

In some embodiments, the subject with the hematologic tumor has not previously received treatment with a BCL-2 inhibitor. In some embodiments, the BCL-2 inhibitor includes, but is not limited to, venetoclax or pelcitoclax.

In some embodiments, optionally, the subject with the hematologic tumor has the following comorbidities:
the tumor patient may be one who has developed an additional malignant tumor within 3 years prior to the first administration or currently has an additional concurrent malignant tumor, provided that the tumor patient has undergone a single surgical treatment for the additional malignant tumor and has achieved 5 consecutive years of disease-free survival (DFS);
or the patient with the tumor or advanced malignant tumor may be a patient with cured cervical carcinoma in situ, non-melanoma skin cancer, and a superficial bladder tumor (including but not limited to Ta (non-invasive tumor), Tis (carcinoma in situ), and T1 (tumor invading the submucosa)).

In some embodiments, the subject with the hematologic tumor does not have any one or more of the following conditions:
1) being a patient diagnosed with Burkitt lymphoma or lymphoblastic lymphoma/leukemia; and/or
2) having central nervous system (CNS) involvement; and/or
3) having previously received allogeneic hematopoietic stem cell transplantation; and/or
4) having an unresolved toxic response of ≥ CTC AE grade 1 due to any previous treatment, excluding alopecia; and/or
5) having active or uncontrolled primary autoimmune cytopenia, including autoimmune hemolytic anemia (AIHA), idiopathic thrombocytopenic purpura (ITP), etc.; and/or
6) having experienced arterial/venous thrombotic events within 6 months prior to the first administration; and/or
7) being a patient with any severe and/or uncontrolled disease; and/or
8) having received chemotherapy or radiotherapy within 4 weeks prior to the first administration, or having received an immune checkpoint inhibitor or CAR-T therapy within 12 weeks prior to the first administration, or having received an additional anti-tumor treatment within 5 half-lives prior to the first administration (with the washout period calculated from the end time of the last treatment); and/or
9) having previously received a BCL-2 inhibitor.

In some embodiments, the arterial/venous thrombotic events occurring within 6 months prior to the first administration described in 6) above optionally refer to cerebrovascular accidents (including but not limited to transient ischemic attack, cerebral hemorrhage, and cerebral infarction), deep vein thrombosis, pulmonary embolism, etc.

In some embodiments, the patient with any severe and/or uncontrolled disease described in 7) above includes: 1. those with myocardial ischemia or myocardial infarction of ≥ grade 2, arrhythmia (QTc > 450 ms for males and QTc > 470 ms for females), and congestive heart failure of ≥ grade 2 (as per the New York Heart Association (NYHA) classification) within 6 months prior to the first administration; 2. those with active severe infection (infection of ≥ CTC AE grade 2), and a left ventricular ejection fraction (LVEF) of < 50% as assessed by cardiac color ultrasound; 3. those with active hepatitis; 4. those with a history of immunodeficiency, including HIV-positive status or other acquired or congenital immunodeficiency diseases, or a history of organ transplantation; 5. those with epilepsy requiring treatment.

### Acute Myeloid Leukemia (AML)

In some embodiments, the acute myeloid leukemia is selected from recurrent and/or refractory acute myeloid leukemia.

In some embodiments, the acute myeloid leukemia is selected from acute myeloid leukemia that is intolerant to a standard therapeutic regimen and has no other better treatment options.

In some embodiments, the patient/subject with acute myeloid leukemia meets one or more of the following conditions:
a) having experienced recurrence within 12 months after achieving CR/CRi and receiving consolidation therapy; and/or
b) having experienced recurrence more than 12 months after achieving CR/CRi but having failed to achieve CR/CRi with re-induction therapy; and/or
c) having experienced recurrence twice or more times; and/or
d) having failed to achieve CR/CRi with first-line induction therapy; and/or
e) being a treatment-naive subject who cannot receive a standard treatment due to reasons such as age, comorbidities, performance status, and/or adverse risk factors.

In some embodiments, the patient with acute myeloid leukemia has experienced recurrence within 12 months after achieving CR/CRi and receiving consolidation therapy.

In some embodiments, the patient with acute myeloid leukemia has experienced recurrence more than 12 months after achieving CR/CRi but has failed to achieve CR/CRi with re-induction therapy.

In some embodiments, the patient with acute myeloid leukemia has experienced recurrence twice or more times.

In some embodiments, the patient with acute myeloid leukemia has failed to achieve CR/CRi with first-line induction therapy.

In some embodiments, the recurrence described in a) above refers to the presence of ≥ 5% peripheral blood leukemic blast cells or bone marrow blast cells, or the emergence of a new extramedullary lesion.

In some embodiments, the failure to achieve CR/CRi with first-line induction therapy described in d) above means that:
for patients eligible for intensive chemotherapy, the first-line standard induction therapy should involve an anthracycline drug and/or standard-dose cytarabine administered for at least 2 cycles;
for patients ineligible for intensive chemotherapy, the first-line induction therapy should involve a demethylating drug administered for at least 4 cycles.
In some embodiments, the acute myeloid leukemia does not include non-acute promyelocytic leukemia.

### Mantle Cell Lymphoma (MCL)

In some embodiments, the lymphoid tumor or non-Hodgkin's lymphoma (NHL) is selected from mantle cell lymphoma.

In some embodiments, the mantle cell lymphoma is selected from recurrent and/or refractory mantle cell lymphoma. In some embodiments, the subject with the mantle cell lymphoma has received treatment with one or two or more prior therapeutic regimens. In some embodiments, the subject with the mantle cell lymphoma has received treatment with one, two, three, four, or five prior therapeutic regimens.

In some embodiments, the subject with the mantle cell lymphoma has previously received at least 1 systemic therapeutic regimen, and has experienced disease progression or intolerance during or after the most recent treatment, or has not achieved a response after adequate treatment.

### Diffuse Large B-Cell Lymphoma (DLBCL)

In some embodiments, the lymphoid tumor or non-Hodgkin's lymphoma is selected from diffuse large B-cell lymphoma.

In some embodiments, the diffuse large B-cell lymphoma is selected from recurrent and/or refractory diffuse large B-cell lymphoma.

In some embodiments, the subject with the diffuse large B-cell lymphoma has received treatment with one or two or more prior therapeutic regimens. In some embodiments, the subject with the diffuse large B-cell lymphoma has received treatment with one, two, three, four, or five prior therapeutic regimens.

In some embodiments, the subject with the diffuse large B-cell lymphoma has previously received at least 1 or 2 systemic therapeutic regimens, and has experienced disease progression or intolerance during or after the most recent treatment, or has not achieved a response after adequate treatment.

### Chronic Lymphocytic Leukemia/Small Lymphocytic Lymphoma (CLL/SLL)

In some embodiments, the lymphoid tumor or non-Hodgkin's lymphoma is selected from the group consisting of chronic lymphocytic leukemia and small lymphocytic lymphoma.

In some embodiments, the chronic lymphocytic leukemia or small lymphocytic lymphoma is selected from recurrent and/or refractory chronic lymphocytic leukemia or small lymphocytic lymphoma.

In some embodiments, the subject with the chronic lymphocytic leukemia or small lymphocytic lymphoma has received treatment with one or two or more prior therapeutic regimens. In some embodiments, the subject with the chronic lymphocytic leukemia or small lymphocytic lymphoma has received treatment with one, two, three, four, or five prior therapeutic regimens.

In some embodiments, the subject with the chronic lymphocytic leukemia or small lymphocytic lymphoma has previously received at least 1 systemic therapeutic regimen, and has experienced disease progression or intolerance during or after the most recent treatment, or has not achieved a response after adequate treatment.

In some embodiments, the subject with the chronic lymphocytic leukemia has a peripheral blood monoclonal B lymphocyte count of > 5 × 10⁹/L, or has a radiologically measurable lesion. In some embodiments, the subject with the chronic lymphocytic leukemia has at least one radiologically measurable tumor lesion in 2 perpendicular directions as assessed by CT or MRI. Optionally, the tumor lesion is selected from the group consisting of a nodal lesion with a longest diameter of > 15 mm and an extranodal lesion with a longest diameter of > 10 mm.

### Administration Regimen

The method of administration can be determined comprehensively based on factors such as the activity and toxicity of the drug, and the tolerance of the subject/patient. Those skilled in the art can determine the appropriate amount, dose, or dosage of each drug used in combination in the present disclosure for administration to the subject/patient. Those skilled in the art can adjust the dose and administration regimen according to methods well known in the art of treatment. For example, the maximum tolerated dose can be readily determined; the effective amount that provides a detectable therapeutic benefit to the subject/patient can also be determined; and the time requirements for administering each drug to provide a detectable therapeutic benefit to the subject/patient can also be determined. Therefore, although the present application exemplifies certain doses and administration regimens, these examples are by no means intended to limit the doses and administration regimens that can be provided to the subject/patient in the practice of the present disclosure.

In some embodiments, the present application provides a method for treating a hematologic tumor, comprising administering to a subject the compound of formula I-1 or the pharmaceutically acceptable salt thereof, or the pharmaceutical composition thereof at a dose (daily or per administration) selected from the group consisting of 20-2000 mg, 20-1200 mg, 100-1200 mg, 100-800 mg, 200-800 mg, and 200-600 mg.

In some embodiments, the present application provides use of the compound of formula I-1 or the pharmaceutically acceptable salt thereof, or the pharmaceutical composition thereof for preparing a medicament for treating a hematologic tumor, wherein the medicament comprises 20-2000 mg, 20-1200 mg, 100-1200 mg, 100-800 mg, 200-800 mg, or 200-600 mg of the compound of formula I-1 or the pharmaceutically acceptable salt thereof, or the pharmaceutical composition thereof. In some embodiments, the present application provides use of the compound of formula I-1 or the pharmaceutically acceptable salt thereof, or the pharmaceutical composition thereof for preparing a medicament for treating a hematologic tumor, wherein the medicament comprises a single dose or multiple doses of the compound of formula I-1 or the pharmaceutically acceptable salt thereof, or the pharmaceutical composition thereof. In some embodiments, the present application provides use of the compound of formula I-1 or the pharmaceutically acceptable salt thereof, or the pharmaceutical composition thereof for preparing a medicament for treating a hematologic tumor, wherein the administration dose (daily or per administration) of the compound of formula I-1 or the pharmaceutically acceptable salt thereof, or the pharmaceutical composition thereof is selected from the group consisting of 20-2000 mg, 20-1200 mg, 100-1200 mg, 100-800 mg, 200-800 mg, and 200-600 mg (e.g., 300 mg, 400 mg, 500 mg, or 600 mg).

The present application further provides the compound of formula I-1 or the pharmaceutically acceptable salt thereof, or the pharmaceutical composition thereof for use in treating a hematologic tumor, wherein the administration dose (daily or per administration) of the compound of formula I-1 or the pharmaceutically acceptable salt thereof, or the pharmaceutical composition thereof is selected from the group consisting of 20-2000 mg, 20-1200 mg, 100-1200 mg, 100-800 mg, 200-800 mg, and 200-600 mg (e.g., 300 mg, 400 mg, 500 mg, or 600 mg).

The present application further provides use of the compound of formula I-1 or the pharmaceutically acceptable salt thereof, or the pharmaceutical composition thereof of the present application for treating a hematologic tumor, wherein the administration dose (daily or per administration) of the compound of formula I-1 or the pharmaceutically acceptable salt thereof, or the pharmaceutical composition thereof is selected from the group consisting of 20-2000 mg, 20-1200 mg, 100-1200 mg, 100-800 mg, 200-800 mg, and 200-600 mg (e.g., 300 mg, 400 mg, 500 mg, or 600 mg).

In some embodiments, the present application further provides a kit for use in treating a tumor, comprising: a pharmaceutical composition comprising the compound of formula I-1 or the pharmaceutically acceptable salt thereof, and optionally further comprising an instruction for use of the pharmaceutical composition of the compound of formula I-1 or the pharmaceutically acceptable salt thereof, wherein the instruction for use involves an administration dose (daily or per administration) of the compound of formula I-1 or the pharmaceutically acceptable salt thereof, or the pharmaceutical composition thereof, selected from the group consisting of 20-2000 mg, 20-1200 mg, 100-1200 mg, 100-800 mg, 200-800 mg, and 200-600 mg (e.g., 300 mg, 400 mg, 500 mg, or 600 mg). In some embodiments, the instruction for use guides the administration of the pharmaceutical composition comprising the compound of formula I-1 or the pharmaceutically acceptable salt thereof to a subject.

In some embodiments, the daily or per-administration dose of the compound of formula I-1 or the pharmaceutically acceptable salt thereof, or the pharmaceutical composition thereof of the present application is selected from the group consisting of 10-5000 mg, 20-5000 mg, 20-4000 mg, 20-3000 mg, 20-2000 mg, 50-1500 mg, 100-1200 mg, 200-1000 mg, 100-1000 mg, 100-800 mg, 200-800 mg, 200-600 mg, and 200-400 mg. In some embodiments, the daily or per-administration dose of the compound of formula I-1 or the pharmaceutically acceptable salt thereof, or the pharmaceutical composition thereof of the present application is selected from the group consisting of 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1100 mg, 1200 mg, 1300 mg, 1400 mg, 1500 mg, 1600 mg, 1700 mg, 1800 mg, 1900 mg, and 2000 mg, and any value within a range formed by any of the above values. In some embodiments, the daily or per-administration dose of the compound of formula I-1 or the pharmaceutically acceptable salt thereof, or the pharmaceutical composition thereof of the present application is selected from the group consisting of 100 mg-1200 mg, 100-800 mg, 200-800 mg, and 200-600 mg. In some embodiments, the daily or per-administration dose of the compound of formula I-1 or the pharmaceutically acceptable salt thereof, or the pharmaceutical composition thereof of the present application is selected from the group consisting of 200-1000 mg and 200-800 mg. In some embodiments, the daily or per-administration dose of the compound of formula I-1 or the pharmaceutically acceptable salt thereof, or the pharmaceutical composition thereof of the present application is selected from the group consisting of 10-800 mg, 20-800 mg, and 100-800 mg. In some embodiments, the daily or per-administration dose of the compound of formula I-1 or the pharmaceutically acceptable salt thereof, or the pharmaceutical composition thereof of the present application is selected from the group consisting of 10-300 mg, 20-400 mg, 20-200 mg, and 20-100 mg. In some embodiments, the daily or per-administration dose of the compound of formula I-1 or the pharmaceutically acceptable salt thereof, or the pharmaceutical composition thereof of the present application is selected from the group consisting of 10 mg, 20 mg, 50 mg, 100 mg, 200 mg, 300 mg, and 400 mg. In some embodiments, the daily or per-administration dose of the compound of formula I-1 or the pharmaceutically acceptable salt thereof, or the pharmaceutical composition thereof of the present application is selected from the group consisting of 200 mg, 300 mg, 400 mg, 500 mg, 600 mg, 800 mg, 1000 mg, and 1200 mg. In some embodiments, the daily or per-administration dose of the compound of formula I-1 or the pharmaceutically acceptable salt thereof, or the pharmaceutical composition thereof of the present application is selected from the group consisting of 100-800 mg. In some embodiments, the daily or per-administration dose of the compound of formula I-1 or the pharmaceutically acceptable salt thereof, or the pharmaceutical composition thereof of the present application is selected from the group consisting of 400-600 mg. In some embodiments, the daily or per-administration dose of the compound of formula I-1 or the pharmaceutically acceptable salt thereof, or the pharmaceutical composition thereof of the present application is selected from the group consisting of 200 mg, 300 mg, 400 mg, 500 mg, and 600 mg.

In some embodiments, the daily or per-administration dose of the compound of formula I-1 or the pharmaceutically acceptable salt thereof, or the pharmaceutical composition thereof of the present application is selected from the group consisting of 50 mg-800 mg. In some embodiments, the daily or per-administration dose of the compound of formula I-1 or the pharmaceutically acceptable salt thereof, or the pharmaceutical composition thereof of the present application is selected from the group consisting of 100 mg-800 mg. In some embodiments, the daily or per-administration dose of the compound of formula I-1 or the pharmaceutically acceptable salt thereof, or the pharmaceutical composition thereof of the present application is selected from the group consisting of 100 mg-600 mg. In some embodiments, the daily or per-administration dose of the compound of formula I-1 or the pharmaceutically acceptable salt thereof, or the pharmaceutical composition thereof of the present application is selected from the group consisting of 50 mg-400 mg (e.g., 50 mg, 100 mg, 200 mg, 300 mg, or 400 mg). In some embodiments, the daily or per-administration dose of the compound of formula I-1 or the pharmaceutically acceptable salt thereof, or the pharmaceutical composition thereof of the present application is selected from the group consisting of 100 mg-400 mg (e.g., 100 mg, 200 mg, or 400 mg). In some embodiments, the daily or per-administration dose of the compound of formula I-1 or the pharmaceutically acceptable salt thereof, or the pharmaceutical composition thereof of the present application is selected from the group consisting of 100 mg-200 mg. In some embodiments, the daily or per-administration dose of the compound of formula I-1 or the pharmaceutically acceptable salt thereof, or the pharmaceutical composition thereof of the present application is selected from the group consisting of 200 mg, 300 mg, 400 mg, 600 mg, and 800 mg.

In some embodiments, the present application provides a method for treating a hematologic tumor, comprising administering to a subject the compound of formula I-1 or the pharmaceutically acceptable salt thereof, or the pharmaceutical composition thereof at a dose (daily or per administration) selected from the group consisting of 20-2000 mg, 20-1200 mg, and 100-1200 mg.

In some embodiments, the present application provides use of the compound of formula I-1 or the pharmaceutically acceptable salt thereof, or the pharmaceutical composition thereof for preparing a medicament for treating a hematologic tumor, wherein the medicament comprises 20-2000 mg, 20-1200 mg, or 100-1200 mg of the compound of formula I-1 or the pharmaceutically acceptable salt thereof, or the pharmaceutical composition thereof. In some embodiments, the present application provides use of the compound of formula I-1 or the pharmaceutically acceptable salt thereof, or the pharmaceutical composition thereof for preparing a medicament for treating a hematologic tumor, wherein the administration dose (daily or per administration) of the compound of formula I-1 or the pharmaceutically acceptable salt thereof, or the pharmaceutical composition thereof is selected from the group consisting of 20-2000 mg, 20-1200 mg, and 100-1200 mg.

The present application further provides the compound of formula I-1 or the pharmaceutically acceptable salt thereof, or the pharmaceutical composition thereof for use in treating a hematologic tumor, wherein the administration dose (daily or per administration) of the compound of formula I-1 or the pharmaceutically acceptable salt thereof, or the pharmaceutical composition thereof is selected from the group consisting of 20-2000 mg, 20-1200 mg, and 100-1200 mg.

The present application further provides use of the compound of formula I-1 or the pharmaceutically acceptable salt thereof, or the pharmaceutical composition thereof of the present application for treating a hematologic tumor, wherein the administration dose (daily or per administration) of the compound of formula I-1 or the pharmaceutically acceptable salt thereof, or the pharmaceutical composition thereof is selected from the group consisting of 20-2000 mg, 20-1200 mg, and 100-1200 mg.

In some embodiments, the present application further provides a kit for use in treating a tumor, comprising: a pharmaceutical composition comprising the compound of formula I-1 or the pharmaceutically acceptable salt thereof, and optionally further comprising an instruction for use of the pharmaceutical composition of the compound of formula I-1 or the pharmaceutically acceptable salt thereof, wherein the instruction for use involves an administration dose (daily or per administration) of the compound of formula I-1 or the pharmaceutically acceptable salt thereof, or the pharmaceutical composition thereof, selected from the group consisting of 20-2000 mg, 20-1200 mg, and 100-1200 mg. In some embodiments, the instruction for use guides the administration of the pharmaceutical composition of the compound of formula I-1 or the pharmaceutically acceptable salt thereof to a subject.

In some embodiments, the daily or per-administration dose of the compound of formula I-1 or the pharmaceutically acceptable salt thereof, or the pharmaceutical composition thereof of the present application is selected from the group consisting of 10-5000 mg, 20-5000 mg, 20-4000 mg, 20-3000 mg, 20-2000 mg, 50-1500 mg, 100-1200 mg, 200-1000 mg, 200-800 mg, 200-600 mg, and 200-400 mg. In some embodiments, the daily or per-administration dose of the compound of formula I-1 or the pharmaceutically acceptable salt thereof, or the pharmaceutical composition thereof of the present application is selected from the group consisting of 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1100 mg, 1200 mg, 1300 mg, 1400 mg, 1500 mg, 1600 mg, 1700 mg, 1800 mg, 1900 mg, and 2000 mg, and any value within a range formed by any of the above values. In some embodiments, the daily or per-administration dose of the compound of formula I-1 or the pharmaceutically acceptable salt thereof, or the pharmaceutical composition thereof of the present application is selected from the group consisting of 100-1200 mg. In some embodiments, the daily or per-administration dose of the compound of formula I-1 or the pharmaceutically acceptable salt thereof, or the pharmaceutical composition thereof of the present application is selected from the group consisting of 200-1000 mg. In some embodiments, the daily or per-administration dose of the compound of formula I-1 or the pharmaceutically acceptable salt thereof, or the pharmaceutical composition thereof of the present application is selected from the group consisting of 10-800 mg and 20 mg-800 mg. In some embodiments, the daily or per-administration dose of the compound of formula I-1 or the pharmaceutically acceptable salt thereof, or the pharmaceutical composition thereof of the present application is selected from the group consisting of 10-300 mg, 20-400 mg, 20-200 mg, and 20-100 mg. In some embodiments, the daily or per-administration dose of the compound of formula I-1 or the pharmaceutically acceptable salt thereof, or the pharmaceutical composition thereof of the present application is selected from the group consisting of 10 mg, 20 mg, 50 mg, 100 mg, 200 mg, 300 mg, and 400 mg. In some embodiments, the daily or per-administration dose of the compound of formula I-1 or the pharmaceutically acceptable salt thereof, or the pharmaceutical composition thereof of the present application is selected from the group consisting of 200 mg, 300 mg, 400 mg, 500 mg, 600 mg, 800 mg, 1000 mg, and 1200 mg. In some embodiments, the daily or per-administration dose of the compound of formula I-1 or the pharmaceutically acceptable salt thereof, or the pharmaceutical composition thereof of the present application is selected from the group consisting of 100-800 mg. In some embodiments, the daily or per-administration dose of the compound of formula I-1 or the pharmaceutically acceptable salt thereof, or the pharmaceutical composition thereof of the present application is selected from the group consisting of 400-600 mg. In some embodiments, the daily or per-administration dose of the compound of formula I-1 or the pharmaceutically acceptable salt thereof, or the pharmaceutical composition thereof of the present application is selected from the group consisting of 200 mg, 300 mg, 400 mg, 500 mg, and 600 mg.

In some embodiments of the present application, the compound of formula I-1 or the pharmaceutically acceptable salt thereof, or the pharmaceutical composition thereof is administered at a dose of 0.0001-20 mg/kg weight per administration. In some embodiments of the present application, the compound of formula I-1 or the pharmaceutically acceptable salt thereof, or the pharmaceutical composition thereof is administered at a dose of 0.0001-20 mg/kg body weight per administration.

In some embodiments, the compound of formula I-1 or the pharmaceutically acceptable salt thereof, or the pharmaceutical composition thereof may be administered at a frequency of thrice daily, twice daily, once daily, once every two days, once every three days, once every four days, once every five days, once every six days, thrice every week, twice every week, once every week, once every two weeks, or once every three weeks. In some embodiments, the compound of formula I-1 or the pharmaceutically acceptable salt thereof, or the pharmaceutical composition thereof may be administered at a frequency of once or twice daily. In some embodiments, the compound of formula I-1 or the pharmaceutically acceptable salt thereof, or the pharmaceutical composition thereof may be administered at a frequency of once daily.

In some embodiments, the administration of the effective amount of the compound of formula I-1 or the pharmaceutically acceptable salt thereof, or the pharmaceutical composition thereof of the present application to the subject is performed daily consecutively.

In some embodiments, the compound of formula I-1 or the pharmaceutically acceptable salt thereof, or the pharmaceutical composition thereof is administered in a single dose or in multiple doses.

In some embodiments, the compound of formula I-1 or the pharmaceutically acceptable salt thereof, or the pharmaceutical composition thereof is administered orally.

In some embodiments, the compound of formula I-1 or the pharmaceutically acceptable salt thereof, or the pharmaceutical composition thereof may be administered orally within 30 min after the start of a meal.

In some embodiments, the compound of formula I-1 or the pharmaceutically acceptable salt thereof, or the pharmaceutical composition thereof is administered orally on an empty stomach.

In some embodiments, 8-32 days constitute 1 administration cycle, for example, 8-28 days, 8-21 days, 8-15 days, 15-28 days, 15-21 days, 21-28 days, 21-32 days, 28-32 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, or 32 days constitute 1 administration cycle. In some embodiments, 28 or 32 days constitute 1 administration cycle. In some embodiments, 28 days (4 weeks) constitute 1 administration cycle. In some embodiments, one or more administration cycles are repeated in the present application.

In some embodiments, the compound of formula I-1 or the pharmaceutically acceptable salt thereof, or the pharmaceutical composition thereof is administered in the following mode: 20 mg-1200 mg, 100 mg-1200 mg, 100 mg-800 mg, 200-800 mg, or 400 mg-600 mg per administration, administered daily (e.g., once daily) consecutively for one or more administration cycles, optionally with 8-32 days (e.g., every 28 days) constituting one administration cycle.

In some embodiments, the compound of formula I-1 or the pharmaceutically acceptable salt thereof, or the pharmaceutical composition thereof is administered in the following mode: 20 mg-1200 mg, 100 mg-1200 mg, 100 mg-800 mg, 200-800 mg, or 400 mg-600 mg per administration, administered daily (e.g., once daily) consecutively for one or more administration cycles, optionally with every 28 days constituting one administration cycle.

In some embodiments, the compound of formula I-1 or the pharmaceutically acceptable salt thereof, or the pharmaceutical composition thereof is administered in the following mode: 100 mg-1200 mg, 100 mg-800 mg, or 200-800 mg per administration, administered orally once daily for one or more administration cycles, optionally with every 28 days constituting one administration cycle.

In some embodiments, the compound of formula I-1 or the pharmaceutically acceptable salt thereof, or the pharmaceutical composition thereof is administered in the following mode: 20-1200 mg, 100-1200 mg, 100-800 mg, 300-600 mg, or 400-600 mg per administration, administered daily (e.g., once daily) consecutively for one or more administration cycles, optionally with every 8-32 days (e.g., every 28 days) constituting one administration cycle.

In some embodiments, the compound of formula I-1 or the pharmaceutically acceptable salt thereof, or the pharmaceutical composition thereof is administered in the following mode: 20-1200 mg, 100-1200 mg, 100-800 mg, 300-600 mg, or 400-600 mg per administration, administered once daily consecutively, optionally with every 28 days constituting one administration cycle.

In some embodiments, the compound of formula I-1 or the pharmaceutically acceptable salt thereof, or the pharmaceutical composition thereof is administered in the following mode: 100-1200 mg or 100-800 mg per administration, administered orally once daily for one or more administration cycles, optionally with every 28 days constituting one administration cycle.

In some embodiments, the compound of formula I-1 or the pharmaceutically acceptable salt thereof, or the pharmaceutical composition thereof may be administered for 2-40 administration cycles, for example, 4-10 or 8-20 administration cycles, or 2, 3, 4, 5, 6, 7, 8, 9, 10, 11, 12, 13, 14, 15, 16, 17, 18, 19, 20, 21, 22, 23, 24, 25, 26, 27, 28, 29, or 30 administration cycles, or even 2-40 or more administration cycles as needed. In some embodiments, the administration of the compound of formula I-1 or the pharmaceutically acceptable salt thereof, or the pharmaceutical composition thereof involves repeating the administration cycle described above until the subject can no longer derive benefit, experiences disease progression, or develops an intolerable toxic response.

In some embodiments, the treatment period of the compound of formula I-1 or the pharmaceutically acceptable salt thereof, or the pharmaceutical composition thereof is 2-40 administration cycles, for example, 4-10 or 8-20 administration cycles, or 2, 3, 4, 5, 6, 7, 8, 9, 10, 11, 12, 13, 14, 15, 16, 17, 18, 19, 20, 21, 22, 23, 24, 25, 26, 27, 28, 29, or 30 administration cycles. In some embodiments, the treatment period of the compound of formula I-1 or the pharmaceutically acceptable salt thereof, or the pharmaceutical composition thereof involves repeating the administration cycle described above until the subject can no longer derive benefit, experiences disease progression, or develops an intolerable toxic response.

In some embodiments, the daily or per-administration dose of the compound of formula I-1 or the pharmaceutically acceptable salt thereof, or the pharmaceutical composition thereof is a treatment period dose.

In some embodiments, the compound of formula I-1 or the pharmaceutically acceptable salt thereof, or the pharmaceutical composition thereof is administered as a single agent; optionally, it may be used in combination with an additional agent.

In some embodiments, in the treatment method described in the present application, a pre-administration escalation period (which may also be referred to as a lead-in period) is also included prior to the administration cycle. In the pre-administration escalation period, the compound of formula I-1 or the pharmaceutically acceptable salt thereof, or the pharmaceutical composition thereof is administered with at least one stepwise escalating dose (hereinafter also referred to as a "step dose"), wherein a first step dose in the at least one step dose is lower than the dose in the administration cycle. In some embodiments, the at least one step dose may comprise the first step dose and an additional step dose. In particular, for a case where the pre-administration escalation period comprises multiple step doses, the last step dose is less than or equal to the dose used in the administration cycle. In a preferred embodiment, the step dose is administered in the same mode and at the same frequency as the dose in the administration cycle. The lead-in period is not counted as part of the administration cycle(s), i.e., the treatment period (the administration dose in the treatment period is the treatment dose).

In some embodiments, for a case where the pre-administration escalation period comprises multiple step doses, the differences between the step doses may be identical or different. In some embodiments, for a case where the pre-administration escalation period comprises multiple step doses, 1-14 step doses, for example, 1, 2, 3, 4, 5, 6, 7, 8, 9, 10, 11, 12, 13, or 14 step doses, may be included.

In some embodiments, the first escalating dose may be selected from the group consisting of: 10 mg, 20 mg, 50 mg, and 100 mg. In a preferred embodiment, each escalating dose may be administered for 1-10 days; for example, it may be administered for 1 day, or administered for 2-10 consecutive days.

In some embodiments, prior to the subject receiving the administration or treatment according to the present application, a daily or weekly stepwise escalating dose of the compound of formula I-1 or the pharmaceutically acceptable salt thereof, or the pharmaceutical composition thereof of the present application is administered in advance to the subject. Optionally, after escalation to a specific dose (e.g., 100 mg, 200 mg, 400 mg, 600 mg, 800 mg, or 1200 mg), the subsequent administration cycle is performed, for example, by daily administration of the compound of formula I-1 or the pharmaceutically acceptable salt thereof, or the pharmaceutical composition thereof at a treatment dose (e.g., 100 mg, 200 mg, 300 mg, 400 mg, 500 mg, 600 mg, 700 mg, 800 mg, or 1200 mg). In some embodiments, for example, the subject has leukemia, for example, acute myeloid leukemia. Herein, the administration of the treatment dose refers to administering the compound of formula I-1 or the pharmaceutically acceptable salt thereof, or the pharmaceutical composition thereof of the present application in the administration cycle in a treatment dose manner at the dose defined above.

In some embodiments, prior to the subject receiving the administration or treatment according to the present application, a daily or weekly stepwise escalating dose of the compound of formula I-1 or the pharmaceutically acceptable salt thereof, or the pharmaceutical composition thereof of the present application is administered in advance to the subject. Optionally, after escalation to a specific dose (e.g., 50 mg, 100 mg, 200 mg, 400 mg, 600 mg, 800 mg, or 1200 mg), the subsequent administration cycle is performed, for example, by daily administration of the compound of formula I-1 or the pharmaceutically acceptable salt thereof, or the pharmaceutical composition thereof at a treatment dose (e.g., 100 mg, 200 mg, 300 mg, 400 mg, 500 mg, 600 mg, 800 mg, or 1200 mg). In some embodiments, the subject has a lymphoid tumor, including non-Hodgkin's lymphoma.

In some embodiments, the administration of the daily stepwise escalating dose is performed for a duration of 2, 3, 4, 5, 6, 7, 8, 9, 10, 11, 12, 13, or 14 days, or a duration within a range formed by any of the above values. In some embodiments, the administration of the daily stepwise escalating dose is performed for a duration of 2-10, 3-4, or 5-7 days.

In some embodiments, optionally, prior to the subject receiving the dose of the administration cycle (e.g., the treatment dose), a stepwise escalating dose of the compound of formula I-1 or the pharmaceutically acceptable salt thereof of the present application is administered to the subject. The stepwise dose escalation comprises changing the dose every 1 day, every 2 days, every 3 days, every 4 days, every 5 days, every 6 days, or every week (7 days) to achieve the stepwise escalating dose. The stepwise dose escalation comprises changing the dose every 1 day to achieve the stepwise escalating dose.

In some embodiments, the stepwise escalating dose comprises at least one of the following: 20 mg, 50 mg, 100 mg, 200 mg, 300 mg, 400 mg, 500 mg, 600 mg, or 800 mg. In some embodiments, the stepwise escalating dose comprises at least one of the following: 20 mg, 50 mg, 100 mg, 200 mg, 400 mg, 600 mg, or 800 mg. In some embodiments, the stepwise escalating dose comprises 20 mg, 50 mg, 100 mg, 200 mg, 300 mg, 400 mg, 500 mg, 600 mg, or 800 mg. In some embodiments, the stepwise escalating dose comprises 20 mg, 50 mg, 100 mg, 200 mg, 400 mg, 600 mg, or 800 mg.

In some embodiments, the first step dose of the stepwise escalating dose is selected from the group consisting of 10 mg, 20 mg, 50 mg, and 100 mg. In some embodiments, a second step dose of the stepwise escalating dose is selected from the group consisting of 20 mg, 50 mg, 100 mg, and 200 mg. Optionally, the stepwise escalating dose further comprises a third step dose. In some embodiments, the third step dose of the stepwise escalating dose is selected from the group consisting of 50 mg, 100 mg, 200 mg, and 400 mg. In some embodiments, optionally, the stepwise escalating dose further comprises a fourth step dose. In some embodiments, optionally, the stepwise escalating dose further comprises a fourth step dose selected from the group consisting of 100 mg, 200 mg, 400 mg, and 600 mg. In some embodiments, optionally, the stepwise escalating dose further comprises a fifth step dose. In some embodiments, optionally, the stepwise escalating dose further comprises a fifth step dose selected from the group consisting of 200 mg, 400 mg, 600 mg, and 800 mg.

In some embodiments, the dose of the administration cycle or the treatment dose comprises 100 mg, 200 mg, 300 mg, 400 mg, 500 mg, 600 mg, 800 mg, or 1200 mg.

In some embodiments, the first step dose of the stepwise escalating dose is selected from 20 mg. In some embodiments, the second step dose of the stepwise escalating dose is selected from 50 mg. Optionally, the stepwise escalating dose further comprises a third step dose. In some embodiments, the third step dose of the stepwise escalating dose is selected from 100 mg. In some embodiments, optionally, the stepwise escalating dose further comprises a fourth step dose. In some embodiments, optionally, the stepwise escalating dose further comprises a fourth step dose selected from 200 mg. In some embodiments, optionally, the stepwise escalating dose further comprises a fifth step dose. In some embodiments, optionally, the stepwise escalating dose further comprises a fifth step dose selected from 400 mg. In some embodiments, optionally, the stepwise escalating dose further comprises a sixth step dose. In some embodiments, optionally, the stepwise escalating dose further comprises a sixth step dose selected from 600 mg. In some embodiments, optionally, the stepwise escalating dose further comprises a seventh step dose. In some embodiments, optionally, the stepwise escalating dose further comprises a seventh step dose selected from 800 mg. In some embodiments, the administration or treatment includes or does not include a lead-in period; for example, when a lead-in period is not included, the treatment period dose is 100 mg.

In some embodiments, the first step dose of the stepwise escalating dose is selected from 100 mg. In some embodiments, the second step dose of the stepwise escalating dose is selected from 200 mg. Optionally, the stepwise escalating dose further comprises a third step dose. In some embodiments, the third step dose of the stepwise escalating dose is selected from 400 mg.

In some embodiments, after the pre-administration escalation period or lead-in period (i.e., after stepwise dose escalation to a specific dose), the compound of formula I-1 or the pharmaceutically acceptable salt thereof, or the pharmaceutical composition thereof is subsequently administered daily in a fixed-dose mode at the dose used in the administration cycle (i.e., at the treatment dose) for treatment. In some embodiments, the dose used in the administration cycle is as defined above; for example, the treatment dose may be selected from the group consisting of 100 mg-400 mg and 100 mg-800 mg. After the second step dose, such as 50 mg, completes the pre-administration escalation period, 100 mg is subsequently administered in a fixed-dose mode in the administration cycle; after the third step dose, such as 100 mg, completes the pre-administration escalation period, 200 mg is subsequently administered in a fixed-dose mode in the administration cycle; for example, after the fourth step dose, such as 200 mg, completes the pre-administration escalation period, 400 mg is subsequently administered in a fixed-dose mode in the administration cycle.

In some embodiments, the treatment dose of the compound of formula I-1 or the pharmaceutically acceptable salt thereof in the administration cycle is selected from the group consisting of 100 mg, 200 mg, 400 mg, 600 mg, 800 mg, and 1200 mg; in some embodiments, it is selected from the group consisting of 100 mg, 200 mg, 400 mg, 600 mg, and 800 mg.

In some embodiments, prior to the subject receiving treatment in the administration cycle (e.g., in a fixed-dose mode), a daily or weekly stepwise escalating dose of the compound of formula I-1 or the pharmaceutically acceptable salt thereof of the present application is administered to the subject. Optionally, after escalation to a specific dose (e.g., 50 mg, 100 mg, 200 mg, 400 mg, 600 mg, 800 mg, or 1200 mg), the subsequent administration cycle is performed, for example, by daily administration of the compound of formula I-1 or the pharmaceutically acceptable salt thereof at a treatment dose (e.g., 100 mg, 200 mg, 400 mg, 600 mg, 800 mg, or 1200 mg).

In some embodiments, prior to the subject receiving treatment in the administration cycle (e.g., in a fixed-dose mode), a daily or weekly stepwise escalating dose of the compound of formula I-1 or the pharmaceutically acceptable salt thereof of the present application is administered to the subject. Optionally, after escalation to a specific dose (e.g., 50 mg, 100 mg, or 200 mg), the subsequent administration cycle is performed, for example, by corresponding daily administration of the compound of formula I-1 or the pharmaceutically acceptable salt thereof at respective treatment doses of 100 mg, 200 mg, and 400 mg. In some embodiments, the daily stepwise escalating doses comprise: administration of 20 mg on the first day, administration of 50 mg on the second day, and administration of 100 mg on the third day. In some embodiments, the daily stepwise escalating doses comprise: administration of 20 mg on the first day, administration of 50 mg on the second day, administration of 100 mg on the third day, and administration of 200 mg on the fourth day. In some embodiments, the daily stepwise escalating doses comprise: administration of 20 mg on the first day, administration of 50 mg on the second day, administration of 100 mg on the third day, administration of 200 mg on the fourth day, and administration of 400 mg on the fifth day. In some embodiments, the daily stepwise escalating doses comprise: administration of 20 mg on the first day, administration of 50 mg on the second day, administration of 100 mg on the third day, administration of 200 mg on the fourth day, administration of 400 mg on the fifth day, and administration of 600 mg on the sixth day.

In some embodiments, the daily stepwise escalating doses comprise: administration of 20 mg on the first day, administration of 50 mg on the second day, administration of 100 mg on the third day, administration of 200 mg on the fourth day, administration of 400 mg on the fifth day, administration of 600 mg on the sixth day, and administration of 800 mg on the seventh day. In some embodiments, the daily stepwise escalating doses comprise: administration of 20 mg on the first day, administration of 50 mg on the second day, administration of 100 mg on the third day, administration of 200 mg on the fourth day, administration of 400 mg on the fifth day, administration of 600 mg on the sixth day, administration of 800 mg on the seventh day, and administration of 1200 mg on the eighth day. In some embodiments, the daily stepwise escalating dose comprises: administration of 100 mg on the first day. In some embodiments, the daily stepwise escalating doses comprise: administration of 100 mg on the first day and administration of 200 mg on the second day. In some embodiments, the daily stepwise escalating doses comprise: administration of 100 mg on the first day, administration of 200 mg on the second day, and administration of 400 mg on the third day. In some embodiments, the daily stepwise escalating doses comprise: administration of 100 mg on the first day, administration of 200 mg on the second day, administration of 400 mg on the third day, and administration of 600 mg on the fourth day. In some embodiments, the daily stepwise escalating doses comprise: administration of 100 mg on the first day, administration of 200 mg on the second day, administration of 400 mg on the third day, and administration of 800 mg on the fourth day.

In some embodiments, the daily stepwise escalating doses comprise: administration of 10 mg on the first day, administration of 20 mg on the second day, administration of 50 mg on the third day, and administration of 100 mg on the fourth day. In some embodiments, the daily stepwise escalating doses comprise: administration of 10 mg on the first day, administration of 20 mg on the second day, administration of 50 mg on the third day, administration of 100 mg on the fourth day, and administration of 200 mg on the fifth day. In some embodiments, the daily stepwise escalating doses comprise: administration of 10 mg on the first day, administration of 20 mg on the second day, administration of 50 mg on the third day, administration of 100 mg on the fourth day, administration of 200 mg on the fifth day, and administration of 300 mg on the sixth day.

In some embodiments, the treatment in the subsequent administration cycle uses the compound of formula I-1 or the pharmaceutically acceptable salt thereof at a treatment dose.

In some embodiments, the daily stepwise escalating dose comprises: administration of 100 mg on the first day; optionally, for the treatment in the subsequent administration cycle, 100 mg or 200 mg is administered daily.

In some embodiments, the daily stepwise escalating doses comprise: administration of 100 mg on the first day and administration of 200 mg on the second day. Optionally, for the treatment in the subsequent administration cycle, 400 mg is administered daily.

In some embodiments, the daily stepwise escalating doses comprise: administration of 100 mg on the first day, administration of 200 mg on the second day, and administration of 400 mg on the third day. Optionally, for the treatment in the subsequent administration cycle, 600 mg or 800 mg is administered daily.

In some embodiments, the daily stepwise escalating doses comprise: administration of 100 mg on the first day, administration of 200 mg on the second day, administration of 400 mg on the third day, and administration of 600 mg on the fourth day. In some embodiments, the daily stepwise escalating doses comprise: administration of 100 mg on the first day, administration of 200 mg on the second day, administration of 400 mg on the third day, and administration of 800 mg on the fourth day.

In some embodiments, the daily stepwise escalating dose comprises: administration of 50 mg on the first day. In some embodiments, the daily stepwise escalating doses comprise: administration of 50 mg on the first day and administration of 100 mg on the second day. In some embodiments, the daily stepwise escalating doses comprise: administration of 50 mg on the first day, administration of 100 mg on the second day, and administration of 200 mg on the third day. In some embodiments, the daily stepwise escalating doses comprise: administration of 50 mg on the first day, administration of 100 mg on the second day, administration of 200 mg on the third day, optionally administration of 300 mg or 400 mg on the fourth day, and optionally administration of 400 mg on the fifth day.

In some embodiments, the administration of the weekly stepwise escalating dose is performed for a duration of 1, 2, 3, 4, 5, 6, or 7 weeks, or a duration within a range formed by any of the above values. In some embodiments, the administration of the weekly stepwise escalating dose is performed for a duration of 2-5, 2-3, or 3-4 weeks. In some embodiments, the administration of the weekly stepwise escalating dose is performed for a duration of 4 weeks.

In some embodiments, the weekly stepwise escalating doses comprise: daily administration of 20 mg in the first week, daily administration of 50 mg in the second week, daily administration of 100 mg in the third week, and daily administration of 200 mg in the fourth week. Optionally, the administration cycle is performed in the fifth week and thereafter, for example, by daily administration at a treatment dose of 400-600 mg (e.g., 400 mg, 500 mg, or 600 mg). In some embodiments, the weekly stepwise escalating doses comprise: daily administration of 10 mg in the first week, daily administration of 20 mg in the second week, daily administration of 50 mg in the third week, daily administration of 100 mg in the fourth week, and daily administration of 200 mg in the fifth week. Optionally, the administration cycle is performed in the sixth week and thereafter, for example, by daily administration at a treatment dose of 200 mg-600 mg (e.g., 200 mg, 300 mg, 400 mg, 500 mg, or 600 mg).

In some embodiments, the daily or weekly stepwise escalating dose of the compound of formula I-1 or the pharmaceutically acceptable salt thereof, or the pharmaceutical composition thereof is the lead-in period dose.

In some embodiments, the method for treating the hematologic tumor provided in the present application comprises:
1) identifying a subject as a patient with the hematologic tumor; and
2) administering to the identified patient a therapeutically effective amount of the compound of formula I-1 or the pharmaceutically acceptable salt thereof, or the pharmaceutical composition thereof.

In some embodiments, the method for treating the hematologic tumor provided in the present application comprises:
1) performing an assay on a sample obtained from a patient to determine whether the patient has the hematologic tumor; and
2) administering to the patient having the hematologic tumor a therapeutically effective amount of the compound of formula I-1 or the pharmaceutically acceptable salt thereof, or the pharmaceutical composition thereof.

In some embodiments, the treatment comprises inhibiting the hematologic tumor in the patient, reducing its severity, decreasing its risk, or suppressing its metastasis.

In some embodiments, the compound of formula I-1 or the pharmaceutically acceptable salt thereof is selected from the compound of formula I or a pharmaceutically acceptable salt thereof. In some embodiments, the compound of formula I-1 or the pharmaceutically acceptable salt thereof is selected from the compound of formula II or a pharmaceutically acceptable salt thereof. In some embodiments, the compound of formula I-1 or the pharmaceutically acceptable salt thereof is selected from the compound of formula III or a pharmaceutically acceptable salt thereof. In some embodiments, the compound of formula I-1 or the pharmaceutically acceptable salt thereof is selected from the compound of formula IV or a pharmaceutically acceptable salt thereof.

Compound of Formula I-1 or Pharmaceutically Acceptable Salt Thereof, or Pharmaceutical Composition Thereof

The compound of formula I-1 of the present application may be administered in its free base form, or in the form of a pharmaceutically acceptable salt, hydrate, or prodrug thereof, wherein the prodrug can be converted *in vivo* into the free base form of the compound of formula I-1.

The pharmaceutical composition of the compound of formula I-1 or the pharmaceutically acceptable salt thereof of the present application comprises the compound of formula I-1 or the pharmaceutically acceptable salt thereof, and further comprises a pharmaceutically acceptable excipient.

In some embodiments, the compound of formula I-1 or the pharmaceutically acceptable salt thereof, or the pharmaceutical composition thereof of the present application is selected from the group consisting of 10-5000 mg, 20-5000 mg, 20-4000 mg, 20-3000 mg, 20-2000 mg, 50-1500 mg, 100-1200 mg, 200-1000 mg, 200-800 mg, 200-600 mg, and 200-400 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1100 mg, 1200 mg, 1300 mg, 1400 mg, 1500 mg, 1600 mg, 1700 mg, 1800 mg, 1900 mg, and 2000 mg, and any value within a range formed by any of the above values, of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 100-1200 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 200-1000 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 10-800 mg and 20 mg-800 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 10-300 mg, 20-400 mg, 20-200 mg, and 20-100 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 10 mg, 20 mg, 50 mg, 100 mg, 200 mg, 300 mg, and 400 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 200 mg, 300 mg, 400 mg, 500 mg, 600 mg, 800 mg, 1000 mg, and 1200 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 100-800 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 300-600 mg and 400-600 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 200 mg, 300 mg, 400 mg, 500 mg, and 600 mg of the compound of formula I-1 or the pharmaceutically acceptable salt thereof, or the pharmaceutical composition thereof.

In some embodiments, the compound of formula I-1 or the pharmaceutically acceptable salt thereof, or the pharmaceutical composition thereof of the present application is selected from the group consisting of 50 mg-800 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 100 mg-800 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 100 mg-600 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 50 mg-400 mg (e.g., 50 mg, 100 mg, 200 mg, 300 mg, or 400 mg) of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 100 mg-400 mg (e.g., 100 mg, 200 mg, or 400 mg) of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 100 mg-200 mg of the compound of formula I-1 or the pharmaceutically acceptable salt thereof, or the pharmaceutical composition thereof. In some embodiments, the compound of formula I-1 or the pharmaceutically acceptable salt thereof, or the pharmaceutical composition thereof of the present application is selected from the group consisting of 200 mg, 400 mg, 600 mg, and 800 mg of the compound of formula I-1 or the pharmaceutically acceptable salt thereof, or the pharmaceutical composition thereof.

In some embodiments, the pharmaceutical composition of the compound of formula I-1 or the pharmaceutically acceptable salt thereof is selected from a solid pharmaceutical composition, including but not limited to a tablet or a capsule.

In some embodiments, the pharmaceutical composition of the compound of formula I-1 or the pharmaceutically acceptable salt thereof is a pharmaceutical composition in a single dose of 5 mg-500 mg, preferably 10 mg-200 mg, and most preferably 10 mg-100 mg. Specifically, it is selected from a pharmaceutical composition in a single dose of 5 mg, 10 mg, 15 mg, 20 mg, 50 mg, 100 mg, 150 mg, 200 mg, 250 mg, 300 mg, 350 mg, 400 mg, 450 mg, or 500 mg, or any value within a range formed by any of the above values. In some embodiments, the single dose of the pharmaceutical composition comprising the compound of formula I-1 or the pharmaceutically acceptable salt thereof is selected from the group consisting of 10 mg, 50 mg, and 100 mg.

In some embodiments, the pharmaceutical composition comprising the compound of formula I-1 or the pharmaceutically acceptable salt thereof is a pharmaceutical composition in multiple doses, which may consist of multiple single doses of the pharmaceutical composition comprising the compound of formula I-1 or the pharmaceutically acceptable salt thereof. In some embodiments, the pharmaceutical composition comprising the compound of formula I-1 or the pharmaceutically acceptable salt thereof is a pharmaceutical composition in multiple doses, which may consist of single doses of 10 mg, 50 mg, or 100 mg of the pharmaceutical composition comprising the compound of formula I-1 or the pharmaceutically acceptable salt thereof.

In some embodiments, the compound of formula I-1 or the pharmaceutically acceptable salt thereof is selected from the compound of formula I or a pharmaceutically acceptable salt thereof. In some embodiments, the compound of formula I-1 or the pharmaceutically acceptable salt thereof is selected from the compound of formula II or a pharmaceutically acceptable salt thereof. In some embodiments, the compound of formula I-1 or the pharmaceutically acceptable salt thereof is selected from the compound of formula III or a pharmaceutically acceptable salt thereof. In some embodiments, the compound of formula I-1 or the pharmaceutically acceptable salt thereof is selected from the compound of formula IV or a pharmaceutically acceptable salt thereof.

### Definitions and Description

Unless otherwise stated, the following terms used in the present application shall have the following meanings. A certain term, unless otherwise specifically defined, should not be considered uncertain or unclear, but interpreted according to its common meaning in the art.

The term "substituted" means that any one or more hydrogen atoms on a specific atom are replaced by a substituent, as long as the valence of the specific atom is normal and the resulting compound is stable. When the substituent is oxo (i.e., =O), it means that two hydrogen atoms are replaced; oxo is not possible on an aromatic group.

The term "optional" or "optionally" means that the subsequently described event or circumstance may, but does not necessarily, occur. The description includes instances where the event or circumstance occurs and instances where it does not. For example, ethyl being "optionally" substituted with halogen means that the ethyl may be unsubstituted (-CH₂CH₃), monosubstituted (e.g., -CH₂CH₂F), polysubstituted (e.g., -CHFCH₂F, -CH₂CHF₂, etc.), or fully substituted (-CF₂CF₃). It will be appreciated by those skilled in the art that for any group comprising one or more substituents, no substitution or substitution pattern that is sterically impossible and/or cannot be synthesized is introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms.

The term "halogen" refers to fluorine, chlorine, bromine, and iodine.

The term "alkyl" refers to hydrocarbyl with a general formula of CₙH₂ₙ₊₁. The alkyl may be linear or branched. For example, the term "C₁₋₆ alkyl" refers to alkyl containing 1 to 6 carbon atoms (e.g., methyl, ethyl, *n*-propyl, isopropyl, *n*-butyl, isobutyl, *see-*butyl, *tert-*butyl*, n*-pentyl, 1-methylbutyl, 2-methylbutyl, 3-methylbutyl, neopentyl, hexyl, 2-methylpentyl, etc.). For another example, the term "C₁₋₄ alkyl" refers to alkyl containing 1 to 4 carbon atoms (e.g., methyl, ethyl, *n*-propyl, isopropyl, *n*-butyl, isobutyl, *sec*-butyl, *tert*-butyl, etc.).

The term "cycloalkyl" refers to a carbocycle that is fully saturated and may exist as a monocyclic ring, a bridged ring, or a spiro ring. Unless otherwise indicated, the carbocycle is typically a 3- to 10-membered ring, preferably a 4- to 6-membered ring. Non-limiting examples of cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, etc.

The term "heterocycloalkyl" refers to a cyclic group that is fully saturated and may exist as a monocyclic ring, a bridged ring, or a spiro ring. Unless otherwise indicated, the heterocycle is typically a 3- to 7-membered ring (e.g., a 4- to 6-membered ring or a 5- to 6-membered ring) containing 1 to 3 (preferably 1 or 2) heteroatoms independently selected from the group consisting of sulfur, oxygen, and/or nitrogen.

Unless otherwise stated, the doses and dose ranges provided in the present application for the compound of formula I-1, such as the compound of formula I, or the pharmaceutically acceptable salt thereof are calculated based on the molecular weight of the free base of the compound of formula I-1, for example, the compound of formula I.

The term "administer", "administration", or "administering" refers to physically introducing a therapeutic agent or a composition comprising the therapeutic agent to an entity using any one of a variety of methods and delivery systems known to those skilled in the art. The terms "administer", "administration", and "administering" are used interchangeably herein.

The term "treat", "treating", or "treatment" usually refers to acquiring a desired pharmacological effect and/or physiological effect. In terms of partially or fully stabilizing or curing a disease and/or a side effect arising from the disease, this effect may be therapeutic. As used herein, "treat", "treating", or "treatment" encompasses any treatment of a disease in a patient, including: (a) inhibiting a symptom of the disease, i.e., blocking the progression of the disease; or (b) alleviating a symptom of the disease, i.e., causing the regression of the disease or the symptom.

The term "effective amount" or "therapeutically effective amount" refers to an amount of the compound of the present application for (i) treating or preventing a specific disease, condition, or disorder; (ii) relieving, ameliorating, or eliminating one or more symptoms of a specific disease, condition, or disorder, or (iii) preventing or delaying the onset of one or more symptoms of the specific disease, condition, or disorder described herein. The amount of the compound of the present application composing the "therapeutically effective amount" varies depending on the compound, the disease state and its severity, the mode of administration, and the age of the mammal to be treated, but may be determined routinely by those skilled in the art in accordance with their knowledge and the present disclosure.

The terms "subject", "patient", and "entity" are used interchangeably herein and refer to an animal, preferably a mammal, preferably a primate (including a human and a non-human primate (e.g., an ape, a monkey, an orangutan, and a chimpanzee, for example, a cynomolgus monkey, a spider monkey, and a macaque, such as a rhesus monkey)), and most preferably a human, that has become the object of treatment, observation, or experimentation. In some embodiments, the subject has experienced and/or exhibited at least one symptom of the disease or disorder to be treated and/or prevented.

As used in the present application, the compound of formula I-1, such as the compound of formula I, or the pharmaceutically acceptable salt thereof may be administered by any applicable route and method, for example, via oral administration or parenteral (e.g., intravenous) administration.

The term "pharmaceutical composition" refers to a mixture consisting of one or more of the compounds or the pharmaceutical combinations thereof or the salts thereof of the present application, together with a pharmaceutically acceptable excipient. The pharmaceutical composition is intended to facilitate the administration of the compound or the pharmaceutical combination thereof of the present application to a subject.

The term "pharmaceutically acceptable excipient" refers to those that do not have a significant irritating effect on an organism and do not impair the biological activity and properties of the active compound. Suitable excipients are well known to those skilled in the art, such as carbohydrates, waxes, water-soluble and/or water-swellable polymers, hydrophilic or hydrophobic materials, gelatin, oils, solvents, water, etc.

The pharmaceutical composition of the present application can be prepared by combining the compound of the present application with a suitable pharmaceutically acceptable excipient, and can be formulated, for example, into a solid formulation such as a tablet, a pill, a capsule, etc.

The pharmaceutical composition of the present application can be manufactured using methods well known in the art, such as conventional mixing, dissolving, granulating, dragee-making, levigating, emulsifying, lyophilizing, etc. The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A solid oral pharmaceutical composition can be prepared by conventional mixing, filling, or tableting. For example, it can be obtained by the following method: mixing the active compound with a solid excipient, optionally grinding the resulting mixture, adding additional suitable excipients if desired, and then processing the mixture into granules to get the core part of a tablet, capsule, or dragee. Suitable excipients include, but are not limited to: binders, diluents, disintegrants, lubricants, glidants, sweeteners, flavoring agents, etc.

The term "pharmaceutically acceptable salt" refers to salts of the compounds of the present application that are within the definition of "pharmaceutically acceptable".

Unless otherwise specified, singular terms encompass plural referents, and vice versa. Unless otherwise specified, the word "a" or "an" refers to "at least one". Unless otherwise stated, "or" is used to mean "and/or".

As used herein, unless otherwise stated, the terms "comprise", "include", and "contain", or equivalents thereof are open-ended expressions, meaning that elements, components, and steps that are not specified may be included in addition to those listed.

The term "single dose" refers to the smallest unit of packaging containing a certain quantity of pharmaceutical product; for example, in a box of seven capsules, each capsule is a single dose; or a vial of injection is a single dose. The term "multiple doses" consists of multiple single doses.

All patents, patent applications, and other identified publications are explicitly incorporated herein by reference for the purpose of description and disclosure. These publications are provided solely because they were disclosed prior to the filing date of the present application. All statements as to the dates of these documents or descriptions as to the content of these documents are based on the information available to the applicant and do not constitute any admission as to the correctness of the dates or the content of these documents. Moreover, in any country or region, any reference to these publications herein should not be construed as an admission that the publications form part of the commonly recognized knowledge in the art.

In one embodiment, the pharmaceutical combination of the present application can be formulated into a pharmaceutical composition that is suitable for a single administration or multiple administrations. In one embodiment, the pharmaceutical combination of the present application may be a pharmaceutical composition in a single dose or in multiple doses.

In the context of cancer, the term "first-line therapy" refers to the first treatment given for a disease. It is usually part of a standard set of treatments, such as post-operative chemotherapy and radiotherapy. The first-line therapy, when used alone, is recognized as the best therapy. If it does not cure the disease or causes serious side effects, other treatment methods may be added or used.

In the context of cancer, the term "second-line therapy" refers to a treatment given when the initial treatment (first-line therapy) is ineffective or ceases to function. The meaning of third-line therapy or multi-line therapy can be deduced accordingly.

The terms "day", "daily", and the like, when referred to in an administration regimen, refer to the time within a calendar day that starts at midnight and ends at the next midnight.

The term "refractory" means that a subject or mammal does not respond to anti-cancer treatment or has an insufficient response to anti-cancer treatment.

The term "adverse event (AE)" refers to any untoward medical occurrence in a subject after receiving an investigational pharmaceutical product, which may manifest as a symptom, sign, disease, or abnormal laboratory examination result, but does not necessarily have a causal relationship with the investigational pharmaceutical product. The evaluation criteria for the nature and severity of adverse events are in accordance with the U.S. National Cancer Institute Common Toxicity Criteria for Adverse Events (NCI-CTC AE v5.0). Items that cannot be evaluated according to these criteria can be evaluated according to the following criteria: Mild: causing only a slight impact on the daily life of the patient. Moderate: causing an obvious impact on the daily life of the patient. Severe: resulting in functional impairment in the patient or causing severe interference with the daily life of the patient.

The term "OS" refers to the overall survival time of a tumor patient.

The term "DFS" refers to the disease-free survival of a tumor patient.

The term "SAE" refers to a serious adverse event.

The term "TEAEs" refers to treatment-related adverse events.

### Technical Effects

The therapeutic regimen of the present application has relatively good efficacy in the treatment of hematologic tumors. The compound of formula I-1, such as the compound of formula I, or the pharmaceutically acceptable salt thereof, or the pharmaceutical composition thereof described in the present application has an excellent effect in at least one of the following aspects: objective response rate (ORR), complete response (CR) rate, median duration of response (DOR), median progression-free survival (PFS), median event-free survival (EFS), and median overall survival (OS), for example, ORR, CR, CRi, partial response (PR), and partial response with lymphocytosis (PR-L). Examples also include CR, CR with minimal residual disease negativity (CR_{MRD-}), CR with incomplete blood cell count recovery (CRi), morphologic leukemia-free state (MLFS), CR with incomplete hematologic recovery (CRh), CR with incomplete platelet recovery (CRp), and PR. Furthermore, the compound of formula I-1, such as the compound of formula I, or the pharmaceutically acceptable salt thereof, or the pharmaceutical composition thereof described in the present application exhibits good drug tolerability in patients, requires a low treatment dose and a short lead-in period, causes minimal side effects (including minimal impact on the cardiovascular function, respiratory system, and central nervous system; for example, the treatment-related adverse events (TRAEs) are primarily hematologic adverse events and abnormal laboratory examination results, mostly of grade 1-2), and can effectively decrease the risk of tumor lysis syndrome (TLS).

This indicates that the compound of formula I-1 (e.g., the compound of formula I) or the pharmaceutically acceptable salt thereof, or the pharmaceutical composition thereof of the present application possesses good pharmaceutical value. Furthermore, it can demonstrate benefits for patients who have previously received treatment (including first-line, second-line, third-line, or fourth-line therapy) or those who have not received treatment. Regarding the criteria for efficacy evaluation: acute myeloid leukemia (AML) may be evaluated with reference to the 2017 European LeukemiaNet (ELN) efficacy evaluation criteria; chronic lymphocytic leukemia (CLL) may be evaluated with reference to the IWCLL 2018 evaluation criteria; small lymphocytic lymphoma (SLL) and other non-Hodgkin's lymphomas (NHLs), such as MCL and DLBCL, may be evaluated with reference to the 2014 Lugano Classification revised criteria.

Efficacy evaluation includes, for example:
Objective response rate (ORR), optionally calculated using the following methods:
SLL, MCL, and DLBCL: the ratio of the number of objective response cases (CR + PR) to the total number of cases;
CLL: the ratio of the number of objective response cases (CR + CRi + PR + PR-L) to the total number of cases;
AML: the ratio of the number of objective response cases (CR + CRi + MLFS + PR) to the total number of cases.
Complete response (CR) rate:
SLL, MCL, and DLBCL: the ratio of the number of complete response cases (CR) to the total number of cases;
CLL: the ratio of the number of complete response cases (CR + CRi) to the total number of cases;
AML: the ratio of the number of complete response cases ((CR + CRi) or (CR + CRh)) to the total number of cases. Median duration of response (DOR): calculated using the Kaplan-Meier method.
Median progression-free survival (PFS) (applicable to NHL), median event-free survival (EFS) (applicable to AML), and median overall survival (OS): estimated using the Kaplan-Meier method.

### DETAILED DESCRIPTION

For clarity, the present application is further described with the following examples, which are, however, not intended to limit the scope of the present application. All reagents used in the present application are commercially available and can be used without further purification.

The compound of formula I-1, such as the compound of formula I, may be prepared with reference to the method disclosed in WO2020088442A1.

### Example 1. Clinical Trial

### 1. Investigational drug

Compound of formula I: strengths: 10 mg and 100 mg, tablets.

### 2. Enrolled patients

### Inclusion criteria:

1) A hematologic malignant tumor definitively diagnosed by histology or cytology;
2) Patient population: non-Hodgkin's lymphoma, etc.; or acute myeloid leukemia, etc.;
3) At least 1 efficacy-evaluable lesion/measurable disease.

### 3. Administration regimen

Tablets of the compound of formula I, administered orally with warm water within 30 min after the start of a meal, at a dose of 20 mg-1200 mg per administration, once daily, with 28 days constituting one administration cycle. The duration of the lead-in period depended on the treatment dose level. The day on which the treatment dose level was reached was designated as C1D1 of the treatment period.

The lead-in period for NHL patients was conducted as follows. All dose groups received once-daily oral administration in a daily escalation manner, with the last dose being the treatment dose:
200 mg dose group: 20 mg → 50 mg → 100 mg → 200 mg;
400 mg dose group: 20 mg → 50 mg → 100 mg → 200 mg → 400 mg;
600 mg dose group: 20 mg → 50 mg → 100 mg → 200 mg → 400 mg → 600 mg;
800 mg dose group: 20 mg → 50 mg → 100 mg → 200 mg → 400 mg → 600 mg → 800 mg;
1200 mg dose group: 20 mg → 50 mg → 100 mg → 200 mg → 400 mg → 600 mg → 800 mg → 1200 mg.

The lead-in period for AML patients was conducted as follows. All dose groups received once-daily oral administration in a daily escalation manner, with the last dose being the treatment dose:
200 mg dose group: 100 mg → 200 mg;
400 mg dose group: 100 mg → 200 mg → 400 mg;
600 mg dose group: 100 mg → 200 mg → 400 mg → 600 mg;
800 mg dose group: 100 mg → 200 mg → 400 mg → 800 mg.

### 4. Efficacy and safety indicators

Efficacy indicators included CR rate, ORR, DOR, PFS, OS, etc.

Adverse events: the occurrence of all adverse events (AEs), serious adverse events (SAEs), and treatment-related adverse events (TEAEs), specifically including, for example, adverse reaction grades, overall incidence of adverse events, etc.

### 5. Trial results

Adverse events were infrequent. The adverse events related to the compound of formula I (TRAEs) were primarily hematologic adverse events and abnormal laboratory examination results, mostly of grade 1-2.

In the AML patients, the ORR was 25%-65%, and the CR rate was 25%-55%. In the 400-600 mg dose groups, the ORR was 56%-70%, and the CR rate was 44%-60%.

In the MCL patients, the ORR was 45%-68%, and the CR rate was 15%-50%. In the 200-600 mg dose groups, the ORR was 45%-68%, and the CR rate was 14%-50%.

In the CLL/SLL patients, the ORR was 89%-95%, and the CR rate was 45%-55%. In the 200-600 mg dose groups, the ORR was 89%-95%, and the CR rate was 44%-55%.

In the DLBCL patients, the ORR was 40%-60%, and the CR rate was 40%-50%. In the 200-600 mg dose groups, the ORR was 40%-60%, and the CR rate was 40%-50%.

In the MCL, CLL/SLL, AML, and DLBCL patients, the ratio of the number of complete response cases/(the number of objective response cases + the number of complete response cases) reached 25%-100%.

### Partial efficacy data:

For example, in the 400-600 mg dose groups, 9 AML patients were evaluated: 4 patients achieved CR/CRi and 1 patient achieved PR, with an ORR of 56% and a CR rate of 44%.

In the 100-600 mg dose groups, 22 MCL patients were evaluated: 3 patients achieved CR and 8 patients achieved PR, with an ORR of 50% and a CR rate of 14%.

In the 200-600 mg dose groups, 27 CLL/SLL patients were evaluated: 12 patients achieved CR/CRi and 12 patients achieved PR, with an ORR of 89% and a CR rate of 44%.

In the 100-600 mg dose groups, 5 DLBCL patients were evaluated: 2 patients achieved CR, with an ORR of 40% and a CR rate of 40%.

For example, in the evaluated MCL patients, the ratio of the number of complete response cases/(the number of objective response cases + the number of complete response cases) reached 27%; in the evaluated CLL/SLL patients, the ratio of the number of complete response cases/(the number of objective response cases + the number of complete response cases) reached 50%; in the evaluated AML patients, the ratio of the number of complete response cases/(the number of objective response cases + the number of complete response cases) reached 71%.

Patients who had previously received treatment (including first-line, second-line, third-line, or fourth-line therapy) or those who had not received treatment could all derive benefits (e.g., they could derive benefits for a relatively long period of time, with good efficacy and low side effects).

Representative cases were as follows:

| Subject No. | Pathological type | Dose group | Previous line of therapy | Best efficacy |
|---|---|---|---|---|
| 1 | MCL | 100 mg | 1 | CR |
| 2 | MCL | 200 mg | 1 | PR |
| 3 | CLL/SLL | 200 mg | 2 | PR |
| 4 | CLL/SLL | 400 mg | 1 | PR |
| 5 | DLBCL | 100 mg | 2 | CR |
| 6 | DLBCL | 400 mg | 1 | CR |
| 7 | AML | 400 mg | 2 | CRi |
| 8 | AML | 400 mg | 1 | CRi |
| 9 | AML | 800 mg | 2 | PR |
| 10 | MCL | 600 mg | 2 | CR |
| 11 | MCL | 400 mg | 3 | CR |
| 12 | MCL | 400 mg | 1 | PR |
| 13 | MCL | 400 mg | 4 | PR |
| 14 | MCL | 600 mg | 4 | PR |
| 15 | CLL/SLL | 400 mg | 1 | CRi |
| 16 | CLL/SLL | 400 mg | 2 | CR |
| 17 | CLL/SLL | 400 mg | 2 | CRi |
| 18 | CLL/SLL | 400 mg | 2 | CR |
| 19 | CLL/SLL | 400 mg | 2 | CR |
| 20 | CLL/SLL | 400 mg | 3 | CR |
| 21 | CLL/SLL | 400 mg | 4 | PR |

## Claims

1. A BCL-2 inhibitor or a pharmaceutically acceptable salt thereof, or a pharmaceutical composition thereof for use in treating a hematologic tumor, the BCL-2 inhibitor or the pharmaceutically acceptable salt thereof being selected from a compound of formula I-1 or a pharmaceutically acceptable salt thereof, 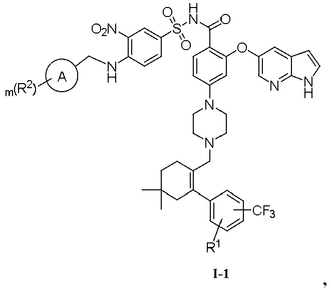
wherein R¹ is selected from the group consisting of hydrogen, halogen, and C₁₋₆ alkyl;
ring A is selected from the group consisting of 5- to 6-membered heterocycloalkyl;
each R² is independently selected from the group consisting of 4- to 6-membered heterocycloalkyl, C₃₋₆ cycloalkyl, -COR^{a}, -SO₂R^{b}, and C₁₋₆ alkyl optionally substituted with halogen;
R^{a} and R^{b} are each independently selected from the group consisting of H, 4- to 6-membered heterocycloalkyl, C₃₋₆ cycloalkyl, and C₁₋₆ alkyl, wherein the C₁₋₆ alkyl is optionally substituted with halogen, CN, -N(C₁₋₆ alkyl)₂, - NHC₁₋₆ alkyl, or -OC₁₋₆ alkyl;
m is selected from the group consisting of 0, 1, 2, and 3, and the hematologic tumor is selected from the group consisting of a lymphoid tumor and myeloid leukemia.

2. The BCL-2 inhibitor or the pharmaceutically acceptable salt thereof, or the pharmaceutical composition thereof according to claim 1, wherein the structural fragment 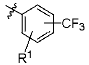 is selected from 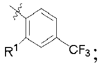
optionally, R¹ is selected from the group consisting of hydrogen, halogen, and C₁₋₃ alkyl;
or R¹ is selected from the group consisting of hydrogen, fluorine, chlorine, and methyl;
or R¹ is selected from the group consisting of hydrogen, chlorine, and methyl;
optionally, ring A is selected from 6-membered heterocycloalkyl;
or ring A is selected from 6-membered heterocycloalkyl containing one or more O atoms;
or ring A is selected from the group consisting of dioxane and a pyran ring;
optionally, each R² is independently selected from the group consisting of 4- to 6-membered heterocycloalkyl, C₄₋₆ cycloalkyl, -COR^{a}, -SO₂R^{b}, and C₁₋₄ alkyl optionally substituted with halogen;
optionally, R^{a} and R^{b} are each independently selected from the group consisting of H, 4- to 6-membered heterocycloalkyl, C₃₋₆ cycloalkyl, and C₁₋₄ alkyl, wherein the C₁₋₄ alkyl is optionally substituted with halogen, CN, -N(C₁₋₄ alkyl)₂, -NHC₁₋₄ alkyl, or -OC₁₋₄ alkyl;
optionally, each R² is independently selected from the group consisting of -C(O)H, -COC(CH₃)₃, -COCF₃, - COCH₂CN, -COCH₂N(CH₃)₂, -SO₂CH₂CH₃, -SO₂CF₃, -SO₂C₂F₅, -CF₃, -C₂F₅, tetrahydropyran, monooxetane, - SO₂-cyclopropane, -CO-cyclopropane, -CO-monooxetane, -SO₂-monooxetane, and -SO₂-cyclobutane;
optionally, m is selected from the group consisting of 0 and 1.

3. The BCL-2 inhibitor or the pharmaceutically acceptable salt thereof, or the pharmaceutical composition thereof according to claim 1 or 2, wherein the BCL-2 inhibitor or the pharmaceutically acceptable salt thereof is selected from the group consisting of the compounds of formula I, formula II, formula III, and formula IV, or pharmaceutically acceptable salts thereof,

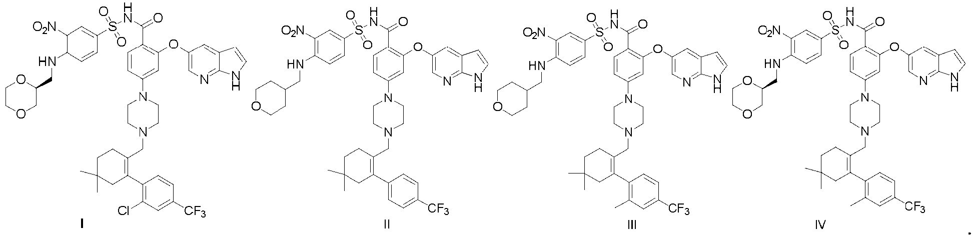

4. The BCL-2 inhibitor or the pharmaceutically acceptable salt thereof, or the pharmaceutical composition thereof according to any one of claims 1-3, wherein
the hematologic tumor is selected from an advanced hematologic tumor;
optionally, the hematologic tumor is selected from a recurrent and/or refractory hematologic tumor;
optionally, the hematologic tumor is selected from a recurrent and/or refractory advanced hematologic tumor;
optionally, the hematologic tumor is selected from a hematologic tumor definitively diagnosed by histology or cytology.

5. The BCL-2 inhibitor or the pharmaceutically acceptable salt thereof, or the pharmaceutical composition thereof according to any one of claims 1-4, wherein the hematologic tumor is selected from the group consisting of non-Hodgkin's lymphoma and acute myeloid leukemia;
optionally, the hematologic tumor is selected from the group consisting of mantle cell lymphoma, diffuse large B-cell lymphoma, chronic lymphocytic leukemia and/or small lymphocytic lymphoma, and acute myeloid leukemia.

6. The BCL-2 inhibitor or the pharmaceutically acceptable salt thereof, or the pharmaceutical composition thereof according to any one of claims 1-5, wherein a patient with the hematologic tumor is a subject who has not received treatment with a prior therapeutic regimen;
optionally, the patient with the hematologic tumor has received treatment with one or two or more prior therapeutic regimens;
optionally, the patient with the hematologic tumor is selected from a subject who has previously received at least 1 systemic therapeutic regimen, and has experienced disease progression or intolerance during or after the most recent treatment, or has not achieved a response after adequate treatment.

7. The BCL-2 inhibitor or the pharmaceutically acceptable salt thereof, or the pharmaceutical composition thereof according to any one of claims 1-6, wherein a daily or per-administration dose of the BCL-2 inhibitor or the pharmaceutically acceptable salt thereof is selected from the group consisting of 20-2000 mg, 20-1200 mg, and 100-1200 mg;
or the daily or per-administration dose is selected from the group consisting of 10-5000 mg, 20-5000 mg, 20-4000 mg, 20-3000 mg, 20-2000 mg, 50-1500 mg, 100-1200 mg, 200-1000 mg, 200-800 mg, 200-600 mg, and 200-400 mg;
or the daily or per-administration dose is selected from the group consisting of 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1100 mg, 1200 mg, 1300 mg, 1400 mg, 1500 mg, 1600 mg, 1700 mg, 1800 mg, 1900 mg, and 2000 mg, and any value within a range formed by any of the above values.

8. The BCL-2 inhibitor or the pharmaceutically acceptable salt thereof, or the pharmaceutical composition thereof according to any one of claims 1-7, wherein the BCL-2 inhibitor or the pharmaceutically acceptable salt thereof is administered at a frequency selected from the group consisting of thrice daily, twice daily, once daily, once every two days, once every three days, once every four days, once every five days, once every six days, thrice every week, twice every week, once every week, once every two weeks, and once every three weeks.

9. The BCL-2 inhibitor or the pharmaceutically acceptable salt thereof, or the pharmaceutical composition thereof according to any one of claims 1-8, wherein the administration of the BCL-2 inhibitor or the pharmaceutically acceptable salt thereof is performed daily consecutively;
optionally, the BCL-2 inhibitor or the pharmaceutically acceptable salt thereof is administered in a single dose or in multiple doses;
optionally, the BCL-2 inhibitor or the pharmaceutically acceptable salt thereof is administered orally;
optionally, the BCL-2 inhibitor or the pharmaceutically acceptable salt thereof is administered orally within 30 min after the start of a meal.

10. The BCL-2 inhibitor or the pharmaceutically acceptable salt thereof, or the pharmaceutical composition thereof according to any one of claims 1-9, wherein prior to the administration, at least one stepwise escalating dose of the BCL-2 inhibitor or the pharmaceutically acceptable salt thereof is administered to the patient, wherein a first stepwise escalating dose is lower than the daily or per-administration dose of the BCL-2 inhibitor or the pharmaceutically acceptable salt thereof used for treatment;
optionally, a daily or weekly stepwise escalating dose of the BCL-2 inhibitor or the pharmaceutically acceptable salt thereof is administered to the patient;
optionally, the at least one stepwise escalating dose comprises the first stepwise escalating dose and an additional stepwise escalating dose, wherein the last stepwise escalating dose is less than or equal to the daily or per-administration dose of the BCL-2 inhibitor or the pharmaceutically acceptable salt thereof used for treatment;
optionally, the administration of the daily stepwise escalating dose is performed for a duration of 2, 3, 4, 5, 6, 7, 8, 9, 10, **11,** 12, 13, or 14 days, or a duration within a range formed by any of the above values.

11. A kit for use in treating a hematologic tumor, comprising: a pharmaceutical composition comprising the BCL-2 inhibitor or the pharmaceutically acceptable salt thereof according to any one of claims 1-10, and optionally further comprising an instruction for use of the pharmaceutical composition of the BCL-2 inhibitor or the pharmaceutically acceptable salt thereof.
